# EUROPEAN PATENT SPECIFICATION

(11) **EP 4 067 504 B1**
(45) Date of publication and mention of the grant of the patent: **17.09.2025**
(21) Application number: 21743632.8
(22) Date of filing: 22.01.2021
(51) Int. Cl.: C12Q 1/68, A61K 38/17, A61P 25/18, A61P 25/24, C12Q 1/6883, G01N 33/50, G01N 33/68

(54) **USE OF SYNAPTOTAGMIN-7 IN THE DIAGNOSIS AND TREATMENT OF BIPOLAR DISORDER**
VERWENDUNG VON SYNAPTOTAGMIN-7 BEI DIAGNOSE UND BEHANDLUNG VON BIPOLAREN STÖRUNGEN
UTILISATION DE SYNAPTOTAGMINE-7 DANS LE DIAGNOSTIC ET LE TRAITEMENT D'UN TROUBLE BIPOLAIRE

(30) Priority: 22.01.2020 CN 202010075636
(43) Date of publication of application: 05.10.2022
(73) Proprietor: Tsinghua University, Beijing 100084 (CN)
(72) Inventor: YAO, Jun, Beijing 100084 (CN); SHEN, Wei, Beijing 100084 (CN); WANG, Qiuwen, Beijing 100084 (CN); LIU, Yaonan, Beijing 100084 (CN)
(74) Representative: Murgitroyd & Company
(86) International application number: PCT/CN2021/073202
(87) International publication number: WO 2021/147977

(56) References cited:
- WO-A1-2008/144371
- WO-A1-2016/181979
- CN-A- 105 142 623
- CN-A- 106 810 602
- CN-A- 108 949 911
- CN-A- 111 172 274
- US-A1- 2010 255 485
- US-A1- 2013 165 336
- US-A1- 2018 120 331
- KANDA MITSURO ET AL: "SYT7 acts as a driver of hepatic metastasis formation of gastric cancer cells", ONCOGENE, NATURE PUBLISHING GROUP UK, LONDON, vol. 37, no. 39, 1 June 2018 (2018-06-01), pages 5355 - 5366, XP036601979, ISSN: 0950-9232, [retrieved on 20180601], DOI: 10.1038/S41388-018-0335-8
- MAXIMOV ANTON ET AL: "Genetic analysis of synaptotagmin-7 function in synaptic vesicle exocytosis", PROCEEDINGS OF THE NATIONAL ACADEMY OF SCIENCES, vol. 105, no. 10, 11 March 2008 (2008-03-11), pages 3986 - 3991, XP093005230, ISSN: 0027-8424, DOI: 10.1073/pnas.0712372105

## Description

### TECHNICAL FIELD

The present invention relates to the field of biopharmaceutics, and in particular to use of synaptotagmin-7 in the diagnosing and treating of bipolar disorder.

### BACKGROUND

Bipolar disorder (BD) based microfluidic systems is a typical neuropsychiatric disorder where patients are afflicted with alternating episodes of two emotional extremes, mania and depression. BD has a global incidence of over 1% and 15% of the patients suicided due to absence of instant intervention. Therefore, BD is listed by the World Health Organization (WHO) as one of the diseases with top morbidity and loss of labor. By genome-wide association analysis, clinical studies have found a series of susceptibility genes for BD that have been thought to be involved in the inheritance and pathogenesis of BD. However, genetic animal models based on such susceptibility genes did not reveal the clinical characteristics of BD, especially the core symptoms of spontaneous alternating cycles of mania and depression. Therefore, a hypothesis has been raised those neuropsychiatric diseases including BD are polygenic genetic diseases, and a joint effort of genetic defects in multiple genes may contribute to the clinical symptoms of the disease. However, patients with a variety of different susceptibility genes can exhibit similar clinical behavioral symptoms as they share common biological defects. Finding and repairing such defects are keys to understanding the pathogenesis of BD and exploring clinical treatment of BD. Thus, identifying the defects of the critical molecular signaling pathways in BD patients is the most important task for biologists and medical professionals studying BD.

Clinical studies have identified several susceptibility genes that may be involved in the inheritance and pathogenesis of BD. For example, a mutant of the CLOCK gene, which was found in a group of BD patients, when expressing in mice, will lead to abnormal behaviors similar to human mania. However, these genes represent only a small fraction of the clinical cases; moreover, genetic animal models based on these genes did not exhibit the spontaneous emotional cycle of mania and depression, which is a core symptom in BD patients.

Therefore, further improvement is required for the research on bipolar disorder.

US2013/165336 discloses an expression biomarker and the use thereof for diagnosing or prognosing Bipolar Disorder.

WO2008/144371 discloses that SYT7 differential expression in **DLPFC** (dorsolateral prefrontal cortex) is also observed in suicide subjects or subjects with mood disorders compared to non suicide subjects.

### SUMMARY

The present invention is intended to solving, at least in part, one of the technical problems in the related art. To this end, one purpose of the present invention is to propose use of synaptotagmin-7 (abbreviated as Syt7) in the diagnosing and treating of bipolar disorder.

The inventor found that Syt7 gene expression in hippocampal neurons differentiated from the induced pluripotent stem cells (iPSCs) of patients with bipolar disorder demonstrated significant defects; Syt7 gene-deleted mice exhibited spontaneous mania/depression cycling symptom of bipolar disorder; the transcription level of Syt7 mRNA in the plasma of patients with BD was significantly reduced as compared with that of a healthy control group; the Syt7 mRNA levels in blood cells were also reduced, similar to the measurements in plasma. Associated signaling pathway with the Syt7 gene and the expression product thereof are critical to the emotional cycle of bipolar disorder. Molecules targeting the signaling pathway associated with the Syt7 gene can facilitate the diagnosis and treatment of bipolar disorder.

Specifically, the present invention provides the following technical scheme:
The invention is as defined in claims 1-5.

Disclosed herein is a biomarker for bipolar disorder, comprising Syt7 gene expression product.

In a first aspect, the present invention provides use of Syt7 gene expression product thereof in preparing a medicament for treating bipolar disorder wherein the medicament is capable of increasing an expression level of the Syt7 gene. According to embodiments of the present invention, the expression product of the Syt7 gene can be used as a protein medicament. By administering a proper amount of the protein medicament to a patient with bipolar disorder or a complication thereof, the content of the Syt7 gene expression product *in vivo* may be elevated. As such, the Syt7 gene expression product can be used for treating bipolar disorder or the complication thereof.

In a second aspect, the present invention provides a bipolar disorder cell model, comprising a reduced amount of Syt7 gene expression product as compared to a wild-type cell.

In a third aspect, the present invention provides a bipolar disorder non-human animal model, comprising a reduced amount of Syt7 gene expression product as compared to a wild-type animal. The expression of Syt7 gene is inhibited in the provided bipolar disorder model animal. According to embodiments of the present invention, the provided bipolar disorder model animal may be a mouse, wherein a C2A domain of the Syt7 gene is replaced with a neomycin gene fragment to achieve the purpose of inactivating the Syt7. It will be appreciated by those skilled in the art that the inactivated Syt7 may also be obtained by substitution or knock-out of other active regions on the Syt7 gene when acquiring the bipolar disorder model animal. The common means may be gene recombination or CRISPR that is conventionally used in the art, and the model animal may be a mouse, a rat, and the like

According to embodiments of the present invention, the animal includes at least one selected from a mouse, and a rat

According to embodiments of the disclosure the determination device further comprises:
the amount of the Syt7 gene expression product in the biological sample being lower than an amount of the Syt7 gene expression product in a normal sample, is an indication that the biological sample has bipolar disorder; or the amount of the Syt7 gene expression product in the biological sample being at least not lower than the amount of the Syt7 gene expression product in the normal sample, is an indication of good prognostic outcome in a patient with bipolar disorder. In an sixth aspect, the present invention provides a method for diagnosing bipolar disorder in a subject, comprising: detecting Syt7 gene expression product in a biological sample from the subject. Also disclosed is a method for diagnosing bipolar disorder in a subject, comprising: detecting an expression level of Syt7 gene in a biological sample from the subject, and determining, if the expression level of the Syt7 gene of the subject is significantly lower than a normal level, that the subject has bipolar disorder.

### BRIEF DESCRIPTION OF THE DRAWINGS

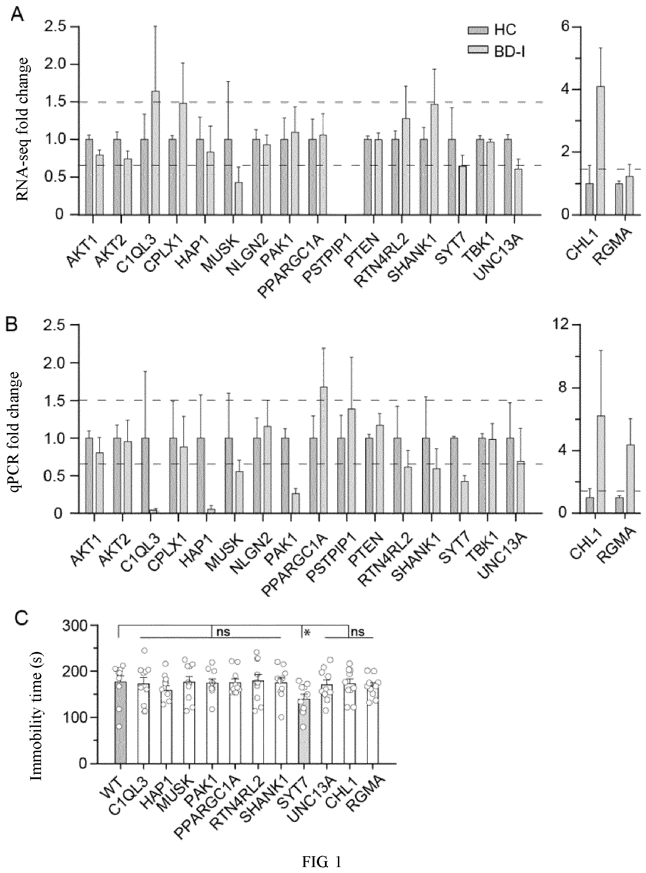

FIG. 1 is a diagram showing the results for identifying that Syt7 participates in behavioral abnormality as a candidate risk factor according to embodiments of the present invention. Panels A and B in FIG. 1 show the expression of 18 candidate genes in hippocampal neurons differentiated from induced pluripotent stem cells (iPSCs), wherein panel A in FIG. 1 shows the result of transcriptome sequencing (RNA-seq), and panel B in FIG. 1 shows the result of fluorescence-based quantitative RT-PCR. Panel C in FIG. 1 shows the immobility time of mice in forced swim test (FST).

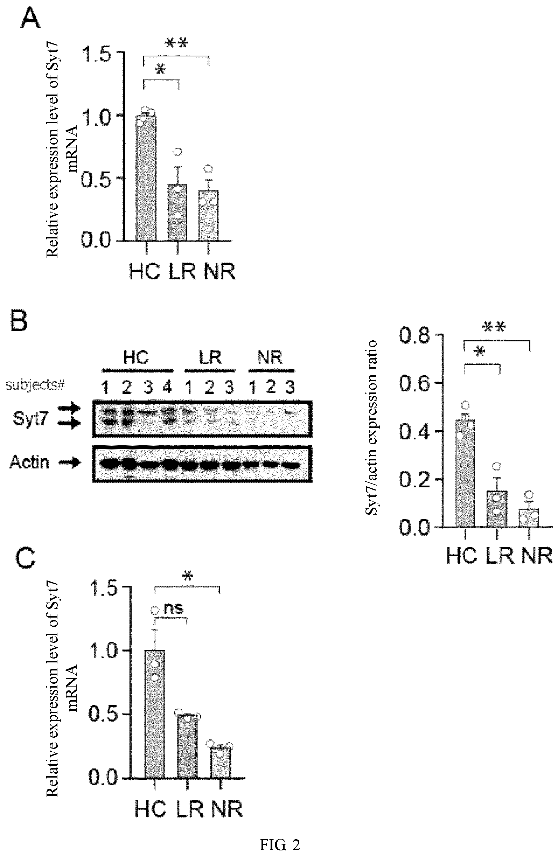

FIG. 2 is a diagram showing the insufficient Syt7 expression in hippocampal neurons differentiated from induced pluripotent stem cells (iPSCs) in patients with confirmed BD according to embodiments of the present invention. Panels A and B in FIG. 2 show the Syt7 expression in one type of hippocampal neurons from patients with LR and NR, wherein panel A in FIG. 2 shows the results of fluorescence quantitative RT-PCR, and panel B in FIG. 2 shows the results of immunoblotting and quantitative analysis, wherein HC denotes the health control group (n = 4), LR denotes lithium-responsive group (n = 3), and NR denotes non-responsive group (n = 3). Panel C in FIG. 2 shows the Syt7 expression in another type of hippocampal neurons from patients with LR and NR, wherein HC denotes the health control group (n = 3), LR denotes lithium-responsive group (n = 3), and NR denotes non-responsive group (n = 3).

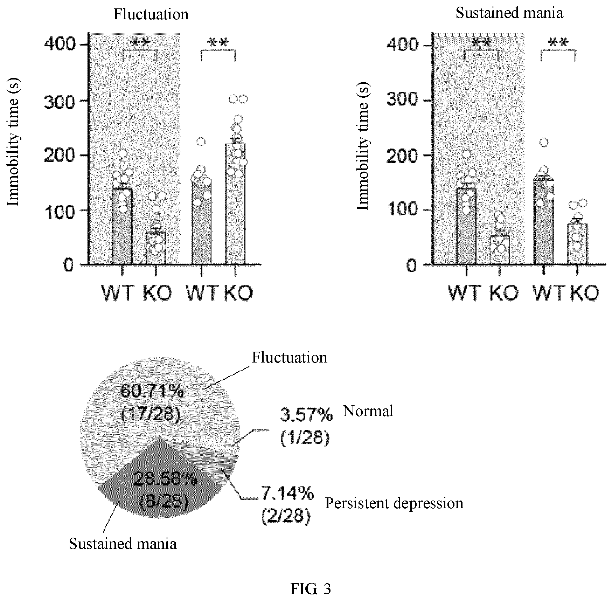

FIG. 3 is a diagram showing the immobility time of wild-type mice and Syt7 KO mice in a night-phase and day-phase forced swim test (FST) according to embodiments of the present invention. The immobility time of Syt7 KO mice shown in FIG. 3 demonstrates a fluctuation in circadian rhythm and a reduction in continuous immobility time of Syt7 KO mice. In FIG. 3, the gray background denotes the night phase, and the white background denotes the day phase; the pie chart shows the proportional phenotype distribution of Syt7 KO mice (n = 28).

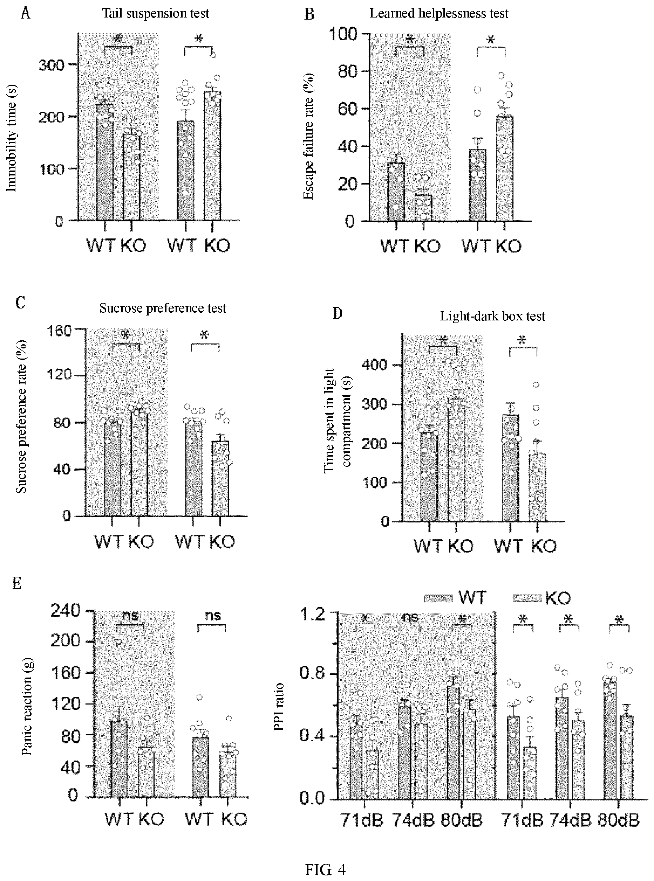

FIG. 4 is a diagram showing the behavioral defects of Syt7 KO mice according to embodiments of the present invention. Panel A in FIG. 4 shows the immobility time of wild-type mice and Syt7 KO mice in a night-phase and day-phase tail suspension (TS) test (n = 12). Panel B in FIG. 4 shows the escape failure number of wild-type mice and Syt7 KO mice in a night-phase and day-phase learned helplessness (LH) test (n = 8). Panel C in FIG. 4 shows the ratio of wild-type mice and Syt7 KO mice demonstrating sucrose preference in a night-phase and day-phase sucrose preference test (SPT, n = 8). Panel D in FIG. 4 shows the time during which the mouse stayed in the light compartment in a night-phase and day-phase light-dark box (LDB) test, wherein for the night phase, n =10, and for the day phase, n = 12. Panel E in FIG. 4 shows the result of a schizophrenia test for Syt7 KO mice.

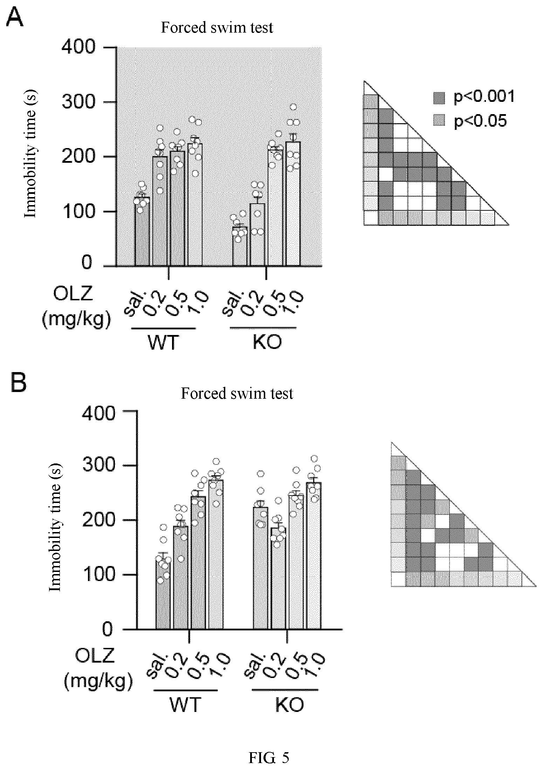

FIG. 5 shows the efficacy of antipsychotic olanzapine and lithium salt on the behavioral abnormality of Syt7 KO mice according to embodiments of the present invention. Panels A and B in FIG. 5 show the effect of olanzapine (OLZ) intraperitoneal injection (0.2-1.0 mg/kg) on the immobility time of mice in a night-phase (A) and day-phase (B) forced swim test (FST).

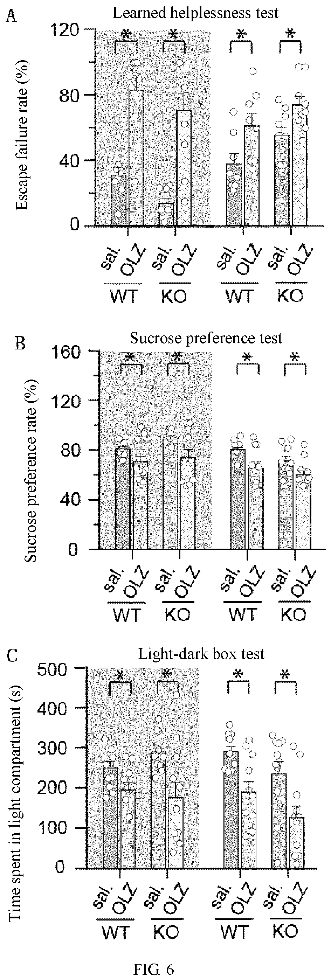

FIG. 6 shows the efficacy of high-dose olanzapine injection (1.0 mg/kg) on behavioral abnormality of Syt7 KO mice according to embodiments of the present invention. Panel A in FIG. 6 shows the effect on the escape failure number in LH test, panel B shows the effect on the sucrose preference in SPT test, and panel C shows the effect on the time spent in the light compartment in LDB test.

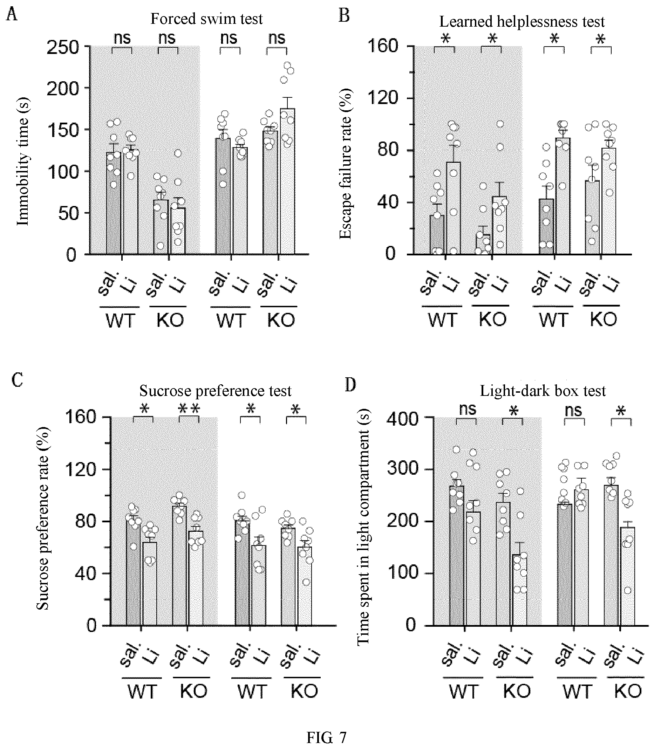

FIG. 7 shows the efficacy of intraperitoneal lithium salt injection (30 mg/kg) on behavioral abnormality of Syt7 KO mice according to embodiments of the present invention. In FIG. 7, panel A shows the effect on the immobility time of mice in FST test, panel B shows the effect on the escape failure number in LH test, panel C shows the effect on the sucrose preference in SPT test, and panel D shows the effect on the time spent in the light compartment in LDB test. In these experiments, n = 8; Student's *t*-test was used; **P* < 0.05; error bars denote s.e.m.

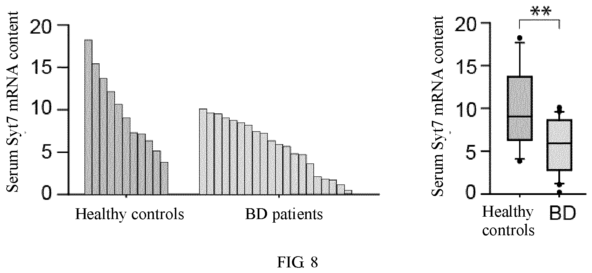

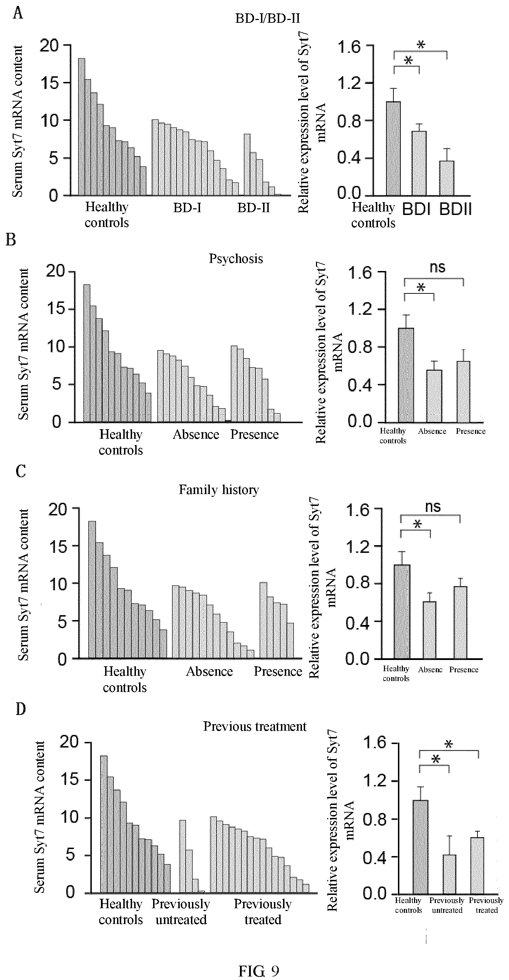

FIG. 8 shows the result of quantitative analytic measurement of plasma Syt7 mRNA level in 20 patients with BD and 11 healthy controls according to embodiments of the present invention. Panel B in FIG. 8 shows the Syt7 mRNA levels in patients with BD-I (n = 11) and BD-II (n = 5). FIG. 9 is a diagram showing results of measurement of plasma Syt7 mRNA level in patients with BD by type of BD, family BD history, previous treatment and presence/absence of psychosis according to embodiments of the present invention. In FIG. 9, panel A shows the level of Syt7 mRNA in patients with BD-I (n = 11) and BD-II (n = 5); panel B shows the comparison of Syt7 mRNA level in BD patients with (n = 7) and without (n = 10) complication (psychosis); panel C shows the comparison of Syt7 mRNA level in BD patients with (n = 5) and without (n = 9) family BD history; panel D shows the comparison of Syt7 mRNA level in BD patients with (n = 14) and without (n = 3) previous treatment. The above results were obtained using Student's *t*-test; **P < 0.05; **P* < 0.001; error bars denote s.e.m.

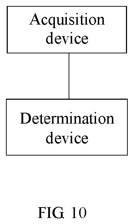

FIG. 10 is a schematic diagram of a system for diagnosing bipolar disorder or determining a prognostic outcome for bipolar disorder according to embodiments of the present invention.

### DETAILED DESCRIPTION

Described herein is a biomarker for bipolar disorder, comprising Syt7 gene and/or an expression product thereof. As used herein, "biomarker" has a general meaning in the art, and refers to a biological molecule and/or a detectable portion thereof, which can be assessed qualitatively or quantitatively to acquire predictive or diagnostic information or the like. For example, the status of a subject with respect to a given disease or condition can be characterized by detecting the corresponding biomarker in the subject. The biomarker may be a gene, a protein, a polypeptide, or the like.

The present invention further provides use of Syt7 gene expression product in preparing a medicament for treating bipolar disorder. According to embodiments of the present invention, the expression product of the Syt7 gene can be used as a protein medicament. By administering a proper amount of the protein medicament to a patient with bipolar disorder the content of the Syt7 gene expression product *in vivo* may be elevated. As such, the Syt7 gene expression product can be used for treating bipolar disorder or the complication thereof.

The present invention further provides a method for diagnosing bipolar disorder in a subject, comprising: detecting Syt7 gene expression product in a biological sample from the subject.

As used herein, reference to a subject or a patient is generally to a human, and may certainly include, as desired, non-human animals, preferably warm-blooded animals, more preferably viviparous animals, more preferably mammals, for example, non-human primates, rodents, canines, felines, equines, sheep, pigs, and the like.

In another aspect, the present disclosure further provides a system for diagnosing bipolar disorder or determining a prognostic outcome for bipolar disorder, as shown in FIG. 10, comprising: an acquisition device for acquiring an amount of Syt7 gene or an expression product thereof in a biological sample; and a determination device connected with the acquisition device for diagnosing bipolar disorder or determining the prognostic outcome for bipolar disorder based on the amount of the Syt7 gene expression product in the biological sample.

According to disclosure the determination device further comprises:
the amount of the Syt7 gene expression product in the biological sample being lower than an amount of the Syt7 gene expression product in a normal sample, is an indication that the biological sample has bipolar disorder; or the amount of the Syt7 gene expression product in the biological sample being at least not lower than the amount of the Syt7 gene expression product in the normal sample, is an indication of good prognostic outcome in a patient with bipolar disorder. According to a preferred embodiment the amount of the Syt7 gene expression product in the biological sample being at least lower than 0.5 folds, preferably 1-fold the amount of the Syt7 gene expression product in a normal sample, is an indication that the biological sample has bipolar disorder.

As used herein, a "sample" or "biological sample" may encompass any biological sample from a subject, including, but not limited to, whole blood, plasma, serum, red blood cells, white blood cells (e.g., peripheral blood mononuclear cells), saliva, urine, stool (i.e., feces), tear, sweat, sebum, nipple aspirate, catheter lavage, tumor exudate, synovial fluid, cerebrospinal fluid, lymphoid fluid, fine needle aspirate, amniotic fluid, any other body fluid, cell lysate, secretion of cells and inflammatory fluid, preferably, plasma, serum or cell pellet.

As used herein, the term "bipolar disorder" is previously known as bipolar disorder in the art. The described bipolar disorder includes bipolar disorder Type I (also referred to as BD-I), bipolar disorder Type II (also referred to as BD-II) and a mixed subtype. Preferably, the Syt7 gene and the expression product thereof can be used as the biomarker of bipolar disorder Type I, and for indicating the presence of bipolar disorder Type I and the prognostic outcome of the medication and the like.

In this context, bipolar disorder Type I is typically characterized by severe mania and severe depression, i.e., similar severities of severe depression and severe mania. Bipolar disorder Type II is typically characterized by hypomania and severe depression, i.e., hypomania with a significantly lower severity than the depressive episode. The mixed subtype mainly refers to an episode during which symptoms of both mania and depression occur simultaneously, and is less common in clinical settings.

As used herein, reference to a complication of bipolar disorder is a disease that results from the development of bipolar disorder, and such a complication may manifest itself as an association with bipolar disorder or as a condition distinct from bipolar disorder, for example, obsessive-compulsive disorder (OCD) with bipolar disorder Type I, which is often accompanied by social fear, anxious (avoidant) personality disorders, rare psychotic symptoms and the like.

The scheme of the present invention as defined by the appended claims will be explained with reference to the following examples
It will be appreciated by those skilled in the art that the following examples are illustrative of the present invention only and should not be construed as limiting the scope of the present invention. Examples without specified techniques or conditions are implemented according to techniques or conditions described in the literature in the art or according to product instructions. Reagents or instruments without specified manufacturers used herein are conventional products that are commercially available.

### Example 1. Syt7 identified as a candidate risk factor for neuropsychiatric disorder-associated behaviors

In order to effectively identify the contributing factors of the emotional abnormality in BD patients, the expression and the function of some genes in iPSC-differentiated neurons from the BD patients were selected and investigated. Also considered was an important feature in BD patients that about 40% of BD patients have disorders of insulin/glucose metabolism (see Ruzickova M, Slaney C, Garnham J, & Alda M, (2003) Clinical Features of Bipolar Disorder with and without Comorbid Diabetes Mellitus, Canadian Journal of Psychiatry, 48(7):458-461; Hajek T, McIntyre R, & Alda M, (2016) Bipolar Disorders, Type 2 Diabetes Mellitus, and the Brain, Current Opinion in Psychiatry, 29(1):1-6; and Wysokinski A, Strzelecki D, & Kloszewska I, (2015) Levels of Triglycerides, Cholesterol, LDL, HDL and Glucose in Patients with Schizophrenia, Unipolar Depression and Bipolar Disorder, Diabetes & Metabolic Syndrome, 9(3):168-176). We supposed, considering that defects in key molecular pathways in the brain will lead to behavioral symptoms, that such defects present in the pancreatic islets may probably cause disorders of insulin metabolism, thereby causing complications of the two.

Based on the above hypothesis, the GeneCards database was searched for keywords insulin (diabetes) and synapse, and 97 genes were retrieved simultaneous participation in insulin metabolism and synaptic function. By searching the NCBI literature (www.ncbi.nlm.nih.gov/pubmed/), 18 out of the 97 genes that may act in the two symptoms above were selected.

Using previously established iPSCs and corresponding differentiated neurons from 6 BD patients (see Mertens J, et al. (2015) Differential Responses to Lithium in Hyperexcitable Neurons from Patients with Bipolar Disorder, Nature, 527(7576):95-99), 11 of the 18 genes in the iPSC-differentiated neurons from the patients exhibited more than 1.5-fold changes in mRNA expression levels by RNA-seq and qRT-PCR approaches (as shown in panels A and B in FIG. 1, wherein the change may be an increase or a decrease). Panel A in FIG. 1 shows the transcriptome sequencing (RNA-seq) result of the 18 candidate genes in hippocampal neurons differentiated from the iPSCs of the BD patients; panel B in FIG. 1 shows the fluorescence-based quantitative RT-PCR result of the 18 candidate genes in hippocampal neurons differentiated from the iPSCs of the BD patients. In panels A and B in FIG. 1, HC denotes healthy controls (n = 4), and BD-I denotes patients with bipolar I disorder (n = 6); the two columns corresponding to each gene along the abscissa respectively denote data for health controls and BD-I patients.

The 11 genes in the DG region of the mice were knocked down using shRNA virus. The mice were subject to a forced swim test (FST), and the immobility time of the mice was analyzed. The immobility time is an indicator that reflects the feeling of despair of the mice, and a longer immobility time indicates a more serious feeling of despair. The result is shown in panel C in FIG. 1. In FIG. 1, panel C shows the results of immobility time of mice in FST test with the candidate genes being knocked-down (KD), wherein n =10 and a t-test was employed (Student's *t*-test; **P* < 0.05; error bars , s.e.m.).

The results showed that the immobility time of the Syt7-knockdown mice was significantly reduced as compared to the wild-type group, and the knockdowns of other genes did not exhibit similar significant changes. Thus, it was presumed that Syt7 may be a possible candidate risk factor for behavioral abnormalities.

### Example 2. Syt7 defect inducing fluctuating behavioral abnormalities in mice

To systematically investigate the role of Syt7 in neuropsychiatric disorder-associated behaviors, the expression of Syt7 in BD patients was studied in two independent iPSC-differentiated neurons of different sources. Panels A and B in FIG. 2 show the expression of Syt7 in hippocampal neurons from patients with LR and NR in Mertens J, et al. (2015) Differential Responses to Lithium in Hyperexcitable Neurons from Patients with Bipolar Disorder, Nature, 527(7576):95-99, wherein LR denotes iPSC-differentiated neurons responsive to lithium (lithium salt) and NR denotes iPSC-differentiated neurons non-responsive to lithium. In FIG. 2, panel C shows Syt7 expression in iPSC-differentiated hippocampal neurons described in Stern S, et al., (2017) Neurons Derived from Patients with Bipolar Disorder Divide into Intrinsically Different Sub-Populations of Neurons, Predicting the Patients' Responsiveness to Lithium, Molecular Psychiatry*.* The results showed that in the two neurons, comparisons with qRT-PCR and immunoblotting analysis detecting the difference from healthy controls demonstrated a reduction in Syt7 expression. This confirmed the presence of Syt7 in BD patients of different subpopulations.

The behaviors of Syt7 KO mice (i.e., Syt7-knockout mice) were then examined in the FST test. It is found that almost 30% of the Syt7 KO mice exhibited shorter immobility time (as the sustained mania results shown in FIG. 3) compared to littermate wild-type mice, whether in the night phase (ZT 12-24) or the day phase (ZT 0-12). Interestingly, over 60% of KO mice exhibited a fluctuating behavioral abnormality phenotype, i.e., a shorter immobility time in the night phase and a longer immobility time in the day phase (as the fluctuation results shown in FIG. 3).

Subsequent behavioral experiments focused on Syt7 KO mice exhibiting the circadian phenotype. In the tail suspension (TS) test, the immobility time of the mice was analyzed. The immobility time is an indicator that reflects the feeling of despair of the mice, and a longer immobility time indicates a more serious feeling of despair. The experimental results showed that the Syt7 KO mice exhibited a significantly shorter immobility time in the night phase and a longer immobility time in the day phase as compared to the wild-type, as shown in panel A in FIG. 4 (in the figures, the gray background denotes the night phase, and the white background denotes the day phase, unless otherwise stated).

Considering that the FST/TS tests can not represent all the characterizations of human disease, a learned helplessness (LH) test and a sucrose preference test (SPT) were conducted. LH is associated with depression. In the LH test, the escape failure number of mice is an indicator for determining the depression of animal due to external pressure. A higher escape failure number indicates a more significant depression. SPT is associated with anhedonia. The proportion of sucrose preference of the mice can serve as an indicator for determining the anhedonia severity of the mice. A higher proportion of sweet preference indicates an enhancement in hedonic feeling. As shown in panels B and C in FIG. 4, Syt7 KO mice were more manic in the night phase (represented by less escape failures in the LH test) and demonstrated higher hedonic feeling in the SPT test compared to wild-type mice; in the day phase, Syt7 KO mice always exhibited the opposite behaviors.

Since BD patients often had clinical symptoms of anxiety, a light-dark box (LDB) test was conducted to observe anxiety-like behaviors in the Syt7 KO mice. The results showed that Syt7 KO mice exhibited reduced anxious behaviors in the night phase and enhanced anxiety in the day phase compared to the wild-type mice, as shown in panel D in FIG. 4.

Meanwhile, in order to confirm whether the behavioral abnormality in Syt7 KO mice is permanent or occurs only in a specific time period, the mice were monitored for 24 h in an open field experiment. As a result, it was found that Syt7 KO mice exhibited an increase in night phase activity but a decrease in day phase activity, which was in correspondence to the results obtained in the FST test and the tail suspension test. Importantly, the changes in activity of the Syt7 KO mice were persistent in the night phase and the day phase, indicating that the emotional abnormalities are persistent.

In addition, since it was found in clinical studies that BD patients often showed symptoms of psychosis, the Syt7 KO mice were observed for psychosis-associated behaviors in a PPI (pre-pulse inhibition) test. The PPI test is a classic behavioral paradigm for determining psychosis in animals. In a PPI test, a lower PPI ratio indicates a more severe psychosis in mice. As shown in panel E in FIG. 4, Syt7 KO mice always showed a lower PPI ratio compared to the wild-type mice either in the night phase or the day phase, indicating a persistent psychotropic behavior in Syt7 KO mice irresponsive to light; furthermore, the Syt7 KO mice showed an audiogenic seizure-like reaction.

In summary, the results showed that the iPSC-differentiated neurons derived from patients with LR and NR have Syt7 defect; more importantly, the Syt7 KO mice exhibited behavioral abnormalities, and exhibited circadian characteristics are unique to the behavioral abnormal fluctuation for bipolar-associated behaviors.

### Example 3. Mood stabilizer treating Syt7 KO mice

To further determine the relationship between behavioral abnormalities and BD characterization in Syt7 KO mice, two drugs, olanzapine (OLZ) and lithium, for clinical and pre-clinical treatment of BD were used to treat Syt7 KO mice and the animal behaviors were observed.

Syt7 KO mice were first treated with the atypical antipsychotic olanzapine (OLZ) and observed for the dose dependence (administered with doses of 0.2 mg/kg, 0.5 mg/kg and 1.0 mg/kg) (using ANOVA statistical method). As shown in panels A and B in FIG. 5, a medium dose of olanzapine was sufficient for altering the behavioral abnormalities in the FST test (i.e., the shorter immobility time in the night phase was reversed) of the Syt7 KO mice in the treated groups as compared to the untreated group; furthermore, the high-dose olanzapine treatment extended the immobility time in the day phase. In addition, treatment with a low dose of olanzapine reduced the immobility time of Syt7 KO mice in the day phase, which was different from the result of the high dose group. Without being limited by theory, this may be associated with an antidepressant effect of olanzapine in treating BD patients.

Then, the LH/SPT/LDB behavioral paradigms were adopted for detecting the effect of high-dose olanzapine treatment (1.0 mg/kg) on abnormal behaviors of the Syt7 KO mice. As shown in panels A, B and C in FIG. 6, high-dose olanzapine treatment alleviated the depressive state of Syt7 KO mice either in the night phase or in the day phase, represented by the significantly increased number of escapes in the LH test, the reduced sucrose preference, and reduced time spent in the light compartment in the LDB test compared to KO mice in the untreated group. In addition, olanzapine treated WT mice showed similar results to the Syt7 KO mice.

The effect of clinical BD treatment lithium on behavioral abnormalities in Syt7 KO mice was then tested. In the FST test for immobility time, the treatment with 30 mg/kg of Li₂CO₃ demonstrated no effect on WT and Syt7 KO mice, either in the night phase or in the day phase, as shown in FIG. 7. However, in both LH and SPT tests, Li-treated WT and Syt7 mice showed improved antidepressant effects, i.e., increased escape numbers and decreased sucrose preference compared to the untreated control group, either in the night phase or in the day phase, as shown in panels B and C in FIG. 7. Furthermore, in the LDB test, the Li treatment had no effect on WT, but altered the behaviors of Syt7 KO mice by reducing the time spent in the light compartment, either in the night phase or in the day phase, as shown in panel D in FIG. 7.

In summary, the results show that the clinical treatments for BD patients have significant efficacies on the behavioral abnormalities of the Syt7 KO mice.

### Example 4. Diagnosis based on Syt7 expression in plasma of BD patients

To explore if the status of Syt7 in BD patients matches defects in iPSC-differentiated neurons, mRNA level in plasma of BD patients was analyzed. Samples from 20 patients with BD and 11 healthy controls (HCs) matched in age and sex were collected and the plasma was separated. The expression level of Syt7 mRNA in plasma was then analyzed by qRT-PCR. The results showed that Syt7 mRNA levels were significantly reduced in the BD group compared to the HC group (as shown in FIG. 8), indicating the presence of Syt7 defect in BD patients.

BD patients were then grouped according to age, sex and severity of the pathological course. Overall, most BD patients showed a decreasing tendency in Syt7 mRNA levels compared to the HC group; it was also noted that the decrease in Syt7 mRNA levels was insignificant in patients aged over 30. Despite the small sample size, the results still suggested that the defect of Syt7 may be more significant in young adults. BD patients were further stratified by type, family BD history, previous treatment and presence of psychosis and analyzed. It was found that the plasma Syt7 mRNA level was significantly reduced in both BD-I and BD-II patients compared to the HC group (as shown in panel A in FIG. 9).

Furthermore, the decrease in Syt7 mRNA level was significant in patients without psychosis and family BD history compared to the HC group, whereas those with symptoms of psychosis or family BD history had a decreasing tendency in Syt7 mRNA (as shown in panels B and C in FIG. 9). It should be noted that patients in the previously untreated group showed a significant decrease in Syt7 mRNA levels despite the small sample size; the previously treated group showed a recovered but still reduced Syt7 mRNA level compared to HC group (as shown in panel D in FIG. 9).

The above experimental results showed that the Syt7 signaling pathway plays a key role in the mood cycling of BD, and developing a diagnostic or treatment method targeting Syt7 upstream and downstream signaling molecules will bring prospect to diagnosis and treatment of BD.

In addition, the terms "first" and "second" are used herein for descriptive purposes only and should not be construed as indicating or implying relative importance or to implicitly indicate the number of technical features described. Thus, a feature defined by "first" or "second" may explicitly or implicitly include at least one feature. In the description "a plurality of" refers to at least two, e.g., two, three, etc., unless explicitly specified otherwise.

In the description of the specification, reference to the description of "one embodiment", "some embodiments", "one example", "one specific example", "some examples" or the like means that the particular features, structures, materials or characteristics described in connection with the embodiment or example is included in at least one embodiment or example of the present disclosure.

In this specification, the schematic representations of the terms used above are not necessarily intended to refer to the same embodiment or example. Furthermore, the particular features, structures, materials or characteristics described may be combined in any suitable manner in any one or more embodiments or examples. Moreover, various embodiments or examples and features of various embodiments or examples described in this specification can be connected and combined by one skilled in the art to the extent that they do not contradict each other.

## Claims

1. A Syt7 gene expression product for use in treating bipolar disorder.

2. Use of a cell model in screening a medicament for treating bipolar disorder, wherein the cell model comprises a reduced amount of Syt7 gene expression product as compared to a wild-type cell.

3. Use of a non-human animal model in screening a medicament for treating bipolar disorder, wherein the animal model comprises a reduced amount of Syt7 gene expression product as compared to a wild-type animal. wherein the animal includes at least one selected from a mouse and a rat.

4. A method for diagnosing bipolar disorder in a subject, comprising: detecting an expression level of the Syt7 gene expression in a biological sample from the subject, and determining, if the expression level of the Syt7 gene of the subject is significantly lower than a normal level, that the subject has bipolar disorder.

## Patentansprüche

1. Ein Syt7-Genexpressionsprodukt zur Verwendung bei der Behandlung bipolarer Störungen.

2. Verwendung eines Zellmodells beim Screening eines Medikaments zur Behandlung bipolarer Störungen, wobei das Zellmodell eine reduzierte Menge an Syt7-Genexpressionsprodukt im Vergleich zu einer Wildtypzelle beinhaltet.

3. Verwendung eines nichtmenschlichen Tiermodells beim Screening eines Medikaments zur Behandlung bipolarer Störung, wobei das Tiermodell eine reduzierte Menge an Syt7-Genexpressionsprodukt im Vergleich zu einem Wildtyptier beinhaltet, wobei das Tier mindestens eines umfasst, das aus einer Maus oder einer Ratte ausgewählt ist.

4. Ein Verfahren zum Diagnostizieren bipolarer Störung bei einem Individuum, das Folgendes beinhaltet: Nachweisen eines Expressionsniveaus der Syt7-Genexpression in einer biologischen Probe des Individuums und Bestimmen, wenn das Expressionsniveau des Syt7-Gens des Individuums bedeutend niedriger ist als ein normales Niveau, dass das Individuum eine bipolare Störung hat.

## Revendications

1. Un produit d'expression du gène Syt7 pour son utilisation dans le traitement d'un trouble bipolaire.

2. Utilisation d'un modèle cellulaire dans le criblage d'un médicament pour le traitement d'un trouble bipolaire, dans lequel le modèle cellulaire comprend une quantité réduite de produit d'expression du gène Syt7 par comparaison avec une cellule de type sauvage.

3. Utilisation d'un modèle animal non-humain dans le criblage d'un médicament pour le traitement d'un trouble bipolaire, dans lequel le modèle animal comprend une quantité réduite de produit d'expression du gène Syt7 par comparaison avec un animal de type sauvage, dans lequel l'animal inclut au moins un animal sélectionné parmi une souris et un rat.

4. Une méthode pour le diagnostic d'un trouble bipolaire chez un sujet, comprenant : la détection d'un niveau d'expression de l'expression du gène Syt7 dans un échantillon biologique prélevé chez le sujet, et la détermination, si le niveau d'expression du gène Syt7 du sujet est significativement plus bas qu'un niveau normal, que le sujet souffre d'un trouble bipolaire.
